# EUROPEAN PATENT APPLICATION

(11) **EP 2 100 964 A1**
(43) Date of publication of application: **16.09.2009**
(21) Application number: 08102478.8
(22) Date of filing: 11.03.2008
(51) Int. Cl.: C12P 7/14, C12P 7/16

(54) **PROCESS FOR THE PRODUCTION OF ETHANOL AND BUTANOL FROM BIOMASS**

(71) Applicant: INEOS EUROPE LIMITED, Lyndhurst Hampshire SO43 7FG (GB)
(72) Inventor: The designation of the inventor has not yet been filed
(74) Representative: King, Alex

(57) **Abstract**

The present invention relates to a process for the production of ethanol and butanol from biomass, and in particular to a process for the production of ethanol and butanol using two separate fermentation steps, said process comprising:
a) passing a biomass feedstock without pasteurisation or sterilisation to a first fermentation step,
b) in said first fermentation step subjecting the biomass feedstock to anaerobic fermentation using a mixture of bacterial strains maintained at conditions to convert the biomass to a product predominantly comprising acetic acid and butyric acid,
c) treating the product stream of step (b) to separate a solution comprising the acetic acid and butyric acid by:
(i) separating a solution comprising the acetic acid and butyric acid from any residual solids and
(ii) separating bacteria and/or pasteurising or sterilising the solution from the first fermentation step,

and
d) in a second fermentation step fermenting the solution comprising the acetic acid and butyric acid from step (c) using a solventogenic bacteria to form ethanol and butanol.

## Description

The present invention relates to a process for the production of ethanol and butanol from biomass, and in particular to a process for the production of ethanol and butanol using two separate fermentation steps.

It has been known for many years that bacteria cause the anaerobic fermentation or digestion of biomass resulting in various gases which can be utilised. As early as the late 19^{th} Century the products of anaerobic digestion were used to generate methane gas for use in street lighting. More recently, anaerobic fermentation has been considered as both a means to reduce the amount of organic matter which is sent to landfill and as an alternative method for production of useful chemicals, such as alcohols.

For example, US 5,753,474 describes a continuous two-stage anaerobic fermentation process to produce butanol using two different strains of bacteria.

The process of US 5,753,474 uses specific strains of bacteria in each stage, and it is necessary to pre-treat the biomass feedstock to sterilise it and kill background bacteria, and thus ensure that the desired bacteria in the first stage can flourish. In such a process the use of a specific strain in the first stage is necessary to obtain high selectivity to a product, such as butyric acid, which is not a "complete" fermentation product. By this it meant that, without the use of a specific selective bacteria as in US 5,753,474, the butyric acid is broken down further to lower acids. In such a process it is also relatively complicated to keep the bacteria alive, which means such processes need significant technical expertise. As an example, it is known that bacteria can be sensitive to butanol inhibition, even to the extent of death of the bacteria being caused by the butanol product. Thus, US 5,753,474 itself notes that it is necessary to keep the product solvents concentration in the solventogenesis reactor below 1.0 wt% to prevent inhibition or even death of the bacteria. However, at such low concentrations it is not economically viable to separate the produced butanol.

For example, in the Encyclopedia of Bioprocess Technology - Fermentation, Biocatalysis, and Bioseparation, Volumes 1-5, p.670-687 the use of clostridium to solventogenise organic acids to the corresponding alcohols is discussed, but it is stated on p.684 that "It has been calculated that when distillation is used in the recovery of ethanol at less than 6-8%, or n-butanol at less than 2 to 3% from fermentation beers, the energy requirement exceeds the net energy recovery in the final products obtained in the commercial process."

We have now found that a significant improvement on the above process can be obtained when producing both ethanol and butanol, with ethanol as the desired predominant final product. In such a case a significant amount of the complexity in the first fermentation step can be avoided because it is not necessary to prevent butyric acid conversion to acetic acid. In fact, such a conversion is desired to ensure that the majority the product acid is acetic acid. The particular advantages of this are that no specific individual bacterial strain is required and that the feed sterilisation taught by US 5,753,474 is not required.

Further, it has been found that the inhibition by product ethanol of the bacteria in the subsequent solventogenesis reaction is not as strong as inhibition by butanol. Therefore, although the concentration of both products should be controlled below certain limits, the total concentration of solvents in the product stream can be significantly higher than is taught by US 5,753,474. This has been found to have significant advantages in allowing economical separation.

Thus, in a first aspect the present invention provides a process for the production of ethanol and butanol using two separate fermentation steps, said process comprising:
a) passing a biomass feedstock without pasteurisation or sterilisation to a first fermentation step,
b) in said first fermentation step subjecting the biomass feedstock to anaerobic fermentation using a mixture of bacterial strains maintained at conditions to convert the biomass to a product predominantly comprising acetic acid and butyric acid,
c) treating the product stream of step (b) to separate a solution comprising the acetic acid and butyric acid by:
   (i) separating a solution comprising the acetic acid and butyric acid from any residual solids and
   (ii) separating bacteria and/or pasteurising or sterilising the solution from the first fermentation step,
   and
d) in a second fermentation step fermenting the solution comprising the acetic acid and butyric acid from step (c) using a solventogenic bacteria to form ethanol and butanol.

The biomass feedstock in step (a) may be any suitable biomass feedstock including, but not limited to, municipal solid waste, lignocellulosic biomass, landfill leachate, carbohydrates, fats and proteins. Specific examples are the biodegradable portion of municipal and industrial wastes, bio-sludge, energy crops and agricultural residues.

The feedstock may be treated by conventional means, such as milling, to make it more easily digested in step (b), but need not be pasteurised or sterilised to remove bacteria therefrom. In fact, as described below, the use of the bacteria inherently present in the biomass in the first fermentation step is an advantageous feature of the present invention.

As used herein, "sterilise" means to treat to effectively kill all bacteria therein. This is typically achieved by application of heat, although other means, such as irradiation, are also known.

As used herein, "pasteurise" means to treat for the purpose of killing bacteria to achieve a 5-log reduction (0.00001 times the original) in the number of live bacteria. Thus, pasteurisation can be distinguished from sterilisation in that some bacteria survive the process. Pasteurisation is typically also performed by the application of heat, generally at lower temperature and/or for a shorter period of time than a corresponding sterilisation. Again, other means, such as irradiation, are also known.

The feedstock pre-treatment is generally selected dependent on the specific feedstock and as necessary or advantageous to make the feedstock more suitable for fermentation. Typically this involves methods to effect size reduction in order to provide improved access for the bacteria and improve the rate of conversion. Examples of known techniques are shredding, milling, ultrasound, hydrocrushing, steam explosion.

In step (b) the biomass feedstock is subjected to anaerobic fermentation using a mixture of bacterial strains maintained at conditions to convert the biomass to a product predominantly comprising acetic acid and butyric acid. In the present invention, the mixture of bacterial strains will include bacterial strains present in the biomass feedstock. In fact, a particular advantage of the process of the present invention is that it can be applied widely to different types of biomass because of the mixture of bacterial strains present.

There are four key stages in normal anaerobic digestion: hydrolysis, acidogenesis, acetogenesis and methanogenesis. Through hydrolysis, complex organic molecules are broken down into simple sugars, acids and amino acids. Bacteria convert these molecules to volatile fatty acids through the process of acidogenesis. In the third stage, acetogenesis of the volatile fatty acids occurs and they are converted to carboxylic acids, such as acetic acid and butyric acid. The final stage in normal anaerobic digestion is methanogenesis, in which acetic acid is broken down to form methane and carbon dioxide.

Generally, the bacteria present in anaerobic digestion can be classified by the final product as either acetogens or methanogens. Both types are likely to be present in the biomass feedstock and in the mixture of bacterial strains in step (b).

In the process of the present invention, the conditions in the first fermentation step are maintained to favour a product predominantly comprising acetic acid and butyric acid, which effectively means conditions that inhibit the methanogenic bacteria. The principal condition necessary for this is the pH, and in the process of the present invention the pH is maintained below 6.0, preferably at a pH in the range 3 to 5.5. At this pH the methanogenic bacteria are inhibited in their activity and reproduction.. Generally, acetogenic bacteria also prefer higher temperatures than methanogenic bacteria. Therefore, whilst temperatures in the range 20 to 70°C may be utilised, preferably the temperature in the first fermentation step is in the range 40 to 60°C, which further inhibits the methanogenic bacteria.

Under such conditions, acid production is favoured whilst production of methane is inhibited. "Wash out" of the methanogens from the bacterial mass can also occur (the draining of methanogenic bacteria through the outlet of the reactor/digester at a faster rate than their generation). Although "higher" acids such as propionic and butyric acids are also produced by digestion of the biomass, these can be further broken down to acetic acid. In contrast, acetic acid is not broken down further e.g. to formic acid and thus, although butyric acid and other "higher" acids are obtained in the present invention they are generally obtained in smaller amounts than the acetic acid. In the process of the present invention, the acetic acid and butyric acid are the predominant products from step (b), by which is meant that these two products are each present in higher concentrations than any other products. Preferably the acetic acid and butyric acid are present in a combined concentration of at least 60 wt%, preferably at least 80 wt% of the total weight of carboxylic acids in the product stream. Acetic acid is the most predominant, and usually the product stream comprises at least a 2:1 ratio of acetic acid to butyric acid.

It has also been found that temperature can be used to control the relative amounts of various acids formed in step (b). In particular, temperatures in the range of 50 to 60°C significantly increase the production of acetic acid over butyric acid and other "higher" acids even compared to lower temperatures in the preferred range of 40 to 60°C, and are thus even more preferred. Under such conditions, the product stream may comprise at least a 4:1 ratio of acetic acid to butyric acid and a combined concentration of the acetic acid and butyric acid of at least 90 wt% of the total weight of carboxylic acids in the product stream.

Nutrients may be added to the first fermentation step as and if required. For example, whilst most manures and complex feedstocks usually inherently contain sufficient nutrients for the bacteria in step (b), other feedstocks, such as industrial wastes and crop residues may be deficient. Typical nutrients requirements include nitrogen, phosphorous, magnesium, sodium, manganese, calcium and cobalt. Nutrients are preferably added by mixture of nutrient rich feedstocks, such as manure, with those that may be nutrient-deficient.

An example of a suitable fermentation process for step (a) is bulk fermentation of a biomass pile as described in US 2006/0024801, but any suitable fermentation tank or vessel may also be used. A number of fermentation tanks/vessels are commercially available, such as the Induced Blanket Reactor available from Andigen LC of Ohio, USA.

Compared to the equivalent step in the process of US 5,753,474 the first fermentation step of the present invention is relatively robust, simple to operate and maintain in continuous operation, and flexible to feedstock changes.

Step (b) produces a product solution comprising acetic acid and butyric acid, bacteria and residual solids which can be removed from the first fermentation step. In step (c) this solution is separated to produce a solution comprising the acetic acid and butyric acid. In step (i) a solution comprising the acetic acid and butyric acid is separated from any residual solids. A suitable means of separation for any residual solids is filtration. In step (ii) bacteria from the first fermentation step are separated and/or the solution is pasteurised or sterilised. For example, bacteria may be separated by filtration with a suitably small mesh filter. Alternatively, or in addition to a filtration to remove bacteria, the solution may be pasteurised or sterilised.

For avoidance of doubt, although steps (a) to (d) of the present invention are performed in the sequence presented, steps (i) and (ii) of step (c) may be performed in any suitable order. Thus, the separation of bacteria and/or pasteurisation or sterilisation of the solution from the first fermentation step may be performed prior to the separation of any residual solids.

The resulting solution is passed to step (d) wherein it is fermented in a second fermentation step using a solventogenic bacteria to form ethanol and butanol.

Any suitable solventogenic bacteria may be used. Particularly suitable examples are *Clostridium acetobutylicum, C. beijerinkii, C. aurantibutyricum, and C. tetanomorphum. Clostridium acetobutylicum* is most preferred.

The solventogenesis process is preferably as described in US 5,753,474.

The ethanol and butanol are obtained from step (d) as a dilute solution in water. Generally, the dilute ethanol and butanol product stream is treated to concentrate and separate the ethanol and butanol with a view to obtaining separate ethanol and butanol streams. The actual final purities of ethanol and butanol desired will depend on the subsequent intended uses, but for ethanol is typically at least 90 wt%, preferably at least 95 wt% and more preferably at least 99 wt%.

The preferred technique for treatment/purification is to use distillation. In a typical distillation column, the butanol may be recovered directly from a side draw on the column. However, ethanol in water cannot be purified by simple distillation to higher than about 95 wt%, at atmospheric pressure, due to the formation of an azeotrope with water. Typically, therefore, the ethanol product is purified to about 90-95 wt% by distillation followed by a drying step, for example on molecular sieve, to give a >99 wt% product. In a preferred embodiment of the present invention, therefore, the initial ethanol and butanol containing product stream from step (d) is treated in a distillation column to produce a butanol containing product stream and an ethanol containing product stream comprising 90 to 95 wt% ethanol, which ethanol containing product stream is subsequently passed to a drying step, especially into contact with a molecular sieve, to remove further water, most preferably to give ethanol in greater than 99 wt% purity.

It is preferable to distil the ethanol to as high a level of purity as possible to reduce the size requirement of the subsequent drying step. However, distilling too close to the azeotrope composition results in an exponential increase in distillation column duty. It is preferable therefore to distil to 94-95 wt% ethanol.

Other alcohols in the product stream may also be removed, as required, by conventional means. Removal from the distillation column as one or more side streams is a preferred example.

The product ethanol and butanol stream from step (d) typically comprises ethanol at a concentration of ethanol in solution of 1 to 5 wt%, more typically in the range 2 to 5 wt%, and butanol at a concentration of butanol in solution of 0.2 to 1 wt%, more typically in the range 0.2 to 0.75 wt%, such as 0.4 to 0.75 wt%.

In particular, when the subsequent treatment/purification comprises distillation, as described above, it is strongly preferred that the initial concentration of ethanol is in the range 2 to 5wt%. Although such solutions are relatively dilute, it has surprisingly been found that only a small penalty in distillation duty is obtained by using initially relatively dilute feeds. In particular, it has been found that when distilling to high purity the rectification operating line is "pinched" close to the vapour-liquid equilibrium curve near to the azeotrope composition. In contrast, it has been found that the operating line at the bottom end of the vapour-liquid equilibrium curve is not pinched unless the concentration of ethanol in the initial stream is below about 2 wt%. For a higher feed concentration of ethanol the pinch remains at the same point at the top of the curve, and the rectification operating line changes minimally. This means that the reflux ratio, and hence the column duty, does not change significantly as the initial feed composition is increased.

For example, it has surprisingly been found that to produce an overheads stream having 94.5 wt% ethanol by distillation from a feed stream with 20 wt% ethanol saves only just over 10% energy compared to producing a stream of the same concentration from a feed stream with only 4 wt% ethanol.

This is in distinct contrast to what has previously been thought in the art, which was that it was not commercially viable to separate ethanol of high purity from initial streams at such low concentrations by distillation. Thus, as noted previously, in the Encyclopedia of Bioprocess Technology - Fermentation, Biocatalysis, and Bioseparation, Volumes 1-5, p.670-687 it was stated that it was not viable to separate ethanol at less than 6 to 8%.

The present invention also overcomes the previous problems of economically separating butanol at low concentrations from fermentation broths. Although the butanol concentration in the product stream from step (d) is below 1 wt%, this can be economically recovered because it is recovered in conjunction with ethanol recovery.

More specifically, it has been found that the butanol may be recovered from the distillation column as a side draw. In particular, a stream having a concentration of butanol of about 40 wt% (of the total stream) may be obtained without a significant increase in column duty. Said stream also comprises smaller amounts of propanol and pentanol (collectively known as fusel oils), and quantities of water and ethanol. This stream may be further treated as required. For example, the stream may be treated to separate the respective alcohols, or the alcohols may be used, after water removal, as a mixed alcohols stream.

It is a distinct advantage to be able to separate ethanol and butanol from such dilute streams in step (d) without significant penalty compared to higher concentrations because the concentrations of acetic acid and butyric acid in the product stream removed from step (b) are also relatively dilute. Thus, the product stream from step (b) preferably has a concentration of acetic acid in solution of 1 to 5 wt%, more typically 2 to 5 wt% and a concentration of butyric acid in solution of 0.2 to 1 wt%, more typically in the range 0.2 to 0.75 wt%, such as 0.4 to 0.75 wt%. The concentration of products in said stream can be controlled by the rate at which the product stream is removed from the fermentation.

As noted previously, at higher concentrations of acetic acid or butyric acid, the acids may inhibit formation of further acid, even to the extent that the acids can kill the bacteria. Although the solution removed from step (b) is relatively dilute, an advantage of the present invention is that no concentration is required before the subsequent second fermentation step and any subsequent separations by distillation.

A number of variations and modifications can be made to the overall process of the present invention. In a particular embodiment a portion of the biomass feed to the first fermentation step or a portion of the feed to the second fermentation step is, instead, passed to a third fermentation step where it is anaerobically digested by a methanogenic bacteria to produce methane, which can be used as a fuel gas and used to meet the energy requirements of the overall process, such as distillation and other separations energy requirements. Use of a portion of the feed to the second fermentation step is preferred in this embodiment since this stream has already been "pre-digested". In addition to meeting the energy requirements of the process, a combined heat and power scheme can be used with the fuel gas to provide electricity (i.e. raise high pressure steam in a boiler, pass the high pressure steam through a steam turbine to convert most of the useful energy into electricity and then use the low pressure steam to provide process heating). Furthermore, waste heat from steam condensate used in the separation section can be used to pre-heat the feed to the fennenters.

### Example

The present invention will be illustrated with respect to the following example using Municipal Solid Waste (MSW) as a biomass feedstock. The MSW is pre-treated by conventional means to make it suitable for anaerobic digestion but is not pasteurised or sterilised.

The feedstock is pre-heated and fed to a continuous fermentation vessel. The fermentation utilises a mixture of acid-producing anaerobic bacteria and the pH of the fermentation is maintained at less than pH 6 to inhibit methanogenesis. The temperature is maintained at about 55°C, which increases the rate of acid production and selectivity to acetic acid rather than higher carboxylic acids, and also helps to inhibit methanogenesis.

Fermented broth containing 2 to 5 wt% acetic acid, 0.2 to 1 wt% butyric acid and small amounts of other carboxylic acids is removed and filtered to remove residual solids. The broth is pasteurised by heating to 72°C. The broth is then passed to a second fermentation vessel. This second fermentation utilises the bacteria *Clostridium acetobutylicum* to convert the carboxylic acids to the relevant alcohols of the same number of carbon atoms.

The product from this step contains 2 to 5 wt% ethanol, 0.2 to 1 wt% butanol and smaller amounts of other alcohols, and is filtered to remove any solids and bacteria. The filtrate is then heated and fed to a distillation column. The distillation bottom product, containing mostly water with small amounts of C5 and above carboxylic acids and solvents, is used to pre-heat the column feed and then recycled to the primary fermentation vessel. To avoid a build up of contaminants in the first fermentation step a small liquid purge may be taken from any suitable point in the process.

A liquid product containing about 40 wt% butanol, along with water, ethanol, propanol and pentanol, is removed as a side draw from the distillation column.

A liquid product containing about 94 wt% ethanol is removed from just below the top of the distillation column. (The liquid product is taken from a number of stages down from the top of the column to allow the removal of light components, including methane, CO2, hydrogen, etc. as a top vapour product from the column.)

The ethanol liquid product is further dehydrated by being contacted with a molecular sieve to give a product >99 wt% ethanol for use as a fuel or chemical feedstock.

## Claims

1. A process for the production of ethanol and butanol using two separate fermentation steps, said process comprising:
a) passing a biomass feedstock without pasteurisation or sterilisation to a first fermentation step,
b) in said first fermentation step subjecting the biomass feedstock to anaerobic fermentation using a mixture of bacterial strains maintained at conditions to convert the biomass to a product predominantly comprising acetic acid and butyric acid,
c) treating the product stream of step (b) to separate a solution comprising the acetic acid and butyric acid by:
(i) separating a solution comprising the acetic acid and butyric acid from any residual solids and
(ii) separating bacteria and/or pasteurising or sterilising the solution from the first fermentation step,
and
d) in a second fermentation step fermenting the solution comprising the acetic acid and butyric acid from step (c) using a solventogenic bacteria to form ethanol and butanol

2. A process according to claim 1 wherein the product stream from step (b) has a concentration of acetic acid in solution of 2 to 5wt% and a concentration of butyric acid in solution of 0.2 to 0.75 wt%.

3. A process according to claim 1 or claim 2 wherein the acetic acid and butyric acid the product stream from step (b) are present in a combined concentration of at least 60 wt% of the total weight of carboxylic acids in the product stream, and the product stream comprises at least a 2:1 ratio of acetic acid to butyric acid.

4. A process according to claim 3 wherein the product stream from step (b) comprises at least a 4:1 ratio of acetic acid to butyric acid and a combined concentration of the acetic acid and butyric acid of at least 90 wt% of the total weight of carboxylic acids in the product stream.

5. A process according to any one of the preceding claims wherein the product ethanol and butanol stream from step (d) comprises ethanol at a concentration of ethanol in solution in the range 2 to 5 wt% and butanol at a concentration of butanol in solution in the range 0.2 to 0.75 wt%.

6. A process according to any one of the preceding claims wherein the mixture of bacterial strains include at least some of the bacteria present in the biomass feedstock.

7. A process according to any one of the preceding claims wherein the pH in step (b) is maintained below 6.0.

8. A process according to any one of the preceding claims wherein the temperature in step (b) is the range 40 to 70°C.

9. A process according to any one of the preceding claims wherein step (c) comprises filtration to remove any residual solids.

10. A process according to any one of the preceding claims wherein step (c) comprises pasteurisation or sterilisation of the solution.

11. A process according to any one of the preceding claims wherein the solventogenic bacteria used in step (d) is selected from *Clostridium acetobutylicum, C. beijerinkii, C. aurantibutyricum, and C. tetanomorphum*.

12. A process according to any one of the preceding claims wherein the biomass is landfill leachate.

13. A process according to any one of the preceding claims wherein a portion of the biomass feed to the first fermentation step or a portion of the feed to the second fermentation step is, instead, passed to a third fermentation step where it is anaerobically digested by a methanogenic bacteria to produce methane.

14. A process according to claim 13 wherein the methane is passed as a fuel gas to a combined heat and power scheme to provide electricity and low pressure steam which are used to meet the energy requirements of the overall process.
